# EUROPEAN PATENT SPECIFICATION

(11) **EP 3 257 451 B1**
(45) Date of publication and mention of the grant of the patent: **04.03.2020**
(21) Application number: 17175995.4
(22) Date of filing: 14.06.2017
(51) Int. Cl.: A61B 17/072

(54) **TOOL ASSEMBLY FOR LEAK RESISTANT TISSUE DISSECTION**
WERKZEUGANORDNUNG ZUR LECKRESISTENTEN DISSEKTION VON GEWEBE
ENSEMBLE D'OUTILS DE DISSECTION DE TISSUS RÉSISTANT AUX FUITES

(30) Priority: 15.06.2016 US 201615182760
(43) Date of publication of application: 20.12.2017
(73) Proprietor: Covidien LP, Mansfield, MA 02048 (US)
(72) Inventor: SGROI, Anthony, Wallingford, CT Connecticut 06492 (US)
(74) Representative: Maschio, Antonio

(56) References cited:
- EP-A2- 0 623 311
- EP-A2- 1 943 960
- EP-A2- 2 959 838
- WO-A1-2008/081210
- US-A1- 2012 080 493

## Description

### BACKGROUND

### 1. Technical Description

The present disclosure is directed to linear surgical stapling devices, and more particularly, to linear surgical stapling devices that include a tool assembly having a longitudinal proximal portion and a curved and/or angled distal portion to facilitate leak resistant dissection of body tissue.

### 2. Background of Related Art

The use of linear surgical stapling devices to perform total or partial removal of a body organ from a patient is known. In some procedures, e.g., gastrectomy procedures, a tool assembly of the surgical stapling device may not be long enough to cut across the entire length of the body organ. In these situations, it is necessary to perform a plurality of cutting operations with the surgical stapling device to remove the organ or a portion of the organ from the patient.

During each cutting operation of a surgical stapling device, tissue is divided along a cut line and staples are applied along the cut line to seal the cut. The end of the cut line defines a termination point having a vertex. Where a surgical procedure requires a plurality of cutting operations to perform the surgical procedure, the cut line includes one or more termination points. Since known surgical staplers do not apply a staple across the vertex of the termination point, the termination point is susceptible to leakage. In order to minimize the likelihood of leakage occurring, the clinician must perform the second cutting operation by intersecting the termination point in a way that the termination point is contained along the side of the organ that is being removed, i.e., the specimen side. Where multiple cutting operations are required, it can be difficult for a clinician to maintain a straight cut line while properly sealing the termination points of the cut line.

Accordingly, a continuing need exists in the art for a linear surgical stapling device that is configured to minimize leakage during procedures that require a plurality of cutting operations. US 2012/080493 A1 describes a surgical instrument including an anvil configured to be translated and/or rotated as the anvil is moved toward a staple cartridge. EP 1943960 B1 describes a surgical stapler having a curved end-effector.

### SUMMARY

The present invention is defined in independent claim 1 and certain optional features thereof are defined in the dependent claims. In so far as the terms "invention", "example", "aspect" and "embodiment" are used herein, this will be interpreted in such a way that the only protection sought is for the invention as claimed. In one aspect of the present disclosure, a tool assembly for leak resistant dissection of tissue includes a cartridge assembly and an anvil. The cartridge defines a first longitudinal axis and includes a linear longitudinal portion and transverse portion that is contiguous with and positioned distally of the longitudinal portion. The cartridge also defines a central knife slot and at least one row of staple receiving pockets positioned on each side of the knife slot. An anvil is positioned along the first longitudinal axis and has a linear longitudinal portion and a transverse portion that is contiguous with and positioned distally of the longitudinal portion. The anvil is supported adjacent to the cartridge assembly and defines an anvil knife slot that is aligned with the central knife slot of the cartridge body and at least one row of staple deforming depressions positioned on each side of the anvil knife slot.

The length of the longitudinal portions of the cartridge and the anvil forms at least 80 percent of the overall length of the tool assembly.

The central knife slot and the at least one row of staple receiving pockets extend along at least a portion of the transverse portion of the cartridge body.

The anvil knife slot and the at least one row of staple deforming depressions extend along at least a portion of the transverse portion of the anvil.

In certain embodiments, the transverse portions of the cartridge and the anvil are curvilinear.

In embodiments, the transverse portions of the cartridge and anvil are curved, definng a radius of curvature.

In some embodiments, the transverse portions of the cartridge and anvil define a second longitudinal axis, the first longitudinal and the second longitudinal axis defining an angle β.

In certain embodiments, angle β is between 15 degrees and 60 degrees.

In embodiments, angle β is between 30 degrees and 45 degrees.

In some embodiments, the distal end of the central knife slot is spaced outwardly from the longitudinal axis a distance X, wherein X is between 2mm and 10mm.

In certain embodiments, X is between 4mm and 8mm.

In another aspect of the present disclosure, a surgical stapling device includes a body portion supporting a tool assembly for leak resistant dissection of tissue that includes a cartridge assembly and an anvil. The cartridge defines a first longitudinal axis and includes a linear longitudinal portion and transverse portion that is contiguous with and positioned distally of the longitudinal portion. The cartridge also defines a central knife slot and at least one row of staple receiving pockets positioned on each side of the knife slot. An anvil is positioned along the first longitudinal axis and has a linear longitudinal portion and a transverse portion that is contiguous with and positioned distally of the longitudinal portion. The anvil is supported adjacent to the cartridge assembly and defines an anvil knife slot that is aligned with the central knife slot of the cartridge body and at least one row of staple deforming depressions positioned on each side of the anvil knife slot.

The length of the longitudinal portions of the cartridge and the anvil can form at least 80 percent of the overall length of the tool assembly.

### BRIEF DESCRIPTION OF THE DRAWINGS

Various embodiments of the presently disclosed tool assembly for leak resistant dissection of tissue are described herein below, as part of a surgical stapling device, with reference to the drawings, wherein:
FIG. 1 is a side perspective view of a surgical stapling device including a powered actuation device and an exemplary embodiment of the presently disclosed tool assembly for leak resistant dissection of tissue;
FIG. 1A is a side perspective view of a surgical stapling device including a manually operated actuation device and the presently disclosed tool assembly shown in FIG. 1;
FIG. 2 is a top view of the tool assembly shown in FIGS. 1 and 2;
FIG. 2A is a top view of the cartridge assembly of the tool assembly shown in FIG. 2 with the anvil removed;
FIG. 2B is a cross-sectional view taken along section line 2B-2B of FIG. 2;
FIG. 2C is a top view of another embodiment of the presently disclosed tool assembly;
FIG. 3 is a top view of tissue after a first cutting operation with the tool assembly shown in FIG. 2;
FIG. 4 is a top view of the tissue after a second cutting operation with the tool assembly shown in FIG. 2; and
FIG. 5 is a top view of the tissue after a third cutting operation with the tool assembly shown in FIG. 2;

### DETAILED DESCRIPTION OF EMBODIMENTS

The presently disclosed tool assembly for leak resistant dissection of tissue will now be described in detail, as part of a surgical stapling device, with reference to the drawings in which like reference numerals designate identical or corresponding elements in each of the several views. In this description, the term "clinician" is used generally to refer to medical personnel including doctors, nurses, and support personnel, the term "proximal" is used generally to refer to the portion of the device that is closer to a clinician, and the term "distal" is used generally to refer to the portion of the device that is farther from the clinician. In addition, the term "endoscopic" is used generally to refer to endoscopic, laparoscopic, arthroscopic, and any other surgical procedure performed through a small incision or a cannula inserted into a patient's body. Finally, the term "specimen side" is used generally to refer to the side or portion of body tissue that is being removed from a patient during a surgical procedure.

The presently disclosed tool assembly is configured to minimize the likelihood of leaks at termination points along a cut line in body tissue. The tool assembly can be integrally secured to a distal end of a surgical stapling device, or in the alternative, form part of a disposable loading unit or reload that is releasably secured to the surgical stapling device. In addition, the configuration of the tool assembly may also be incorporated into both endoscopic and open-type surgical stapling devices.

The tool assembly includes an anvil and a cartridge housing a plurality of staples. The tool assembly, including each of the anvil and cartridge has a linear longitudinal proximal portion and a transverse distal portion. The linear proximal portion is substantially longer than the transversely extending distal portion. In embodiments, the distal portion of the tool assembly may be curved or define an angle with the linear proximal portion. In use, when a surgical procedure is performed that requires a plurality of cutting operations, e.g., a gastrectomy procedure, the first cutting operation is performed to form a substantially linear cut line having a curved distal portion that curves outwardly towards a specimen side of the tissue being operated on, e.g., the stomach. In each subsequent cutting operation, the proximal portion of the tool assembly is positioned in overlapping relation with the end of a linear portion of the existing cut line with a termination point of the existing cut line being curved or angled towards the specimen side of the tissue being partially removed. By positioning the termination points on the specimen side of the organ being operated upon, the termination points of the cut line are positioned on the specimen side and are removed from the patient with the tissue being removed.

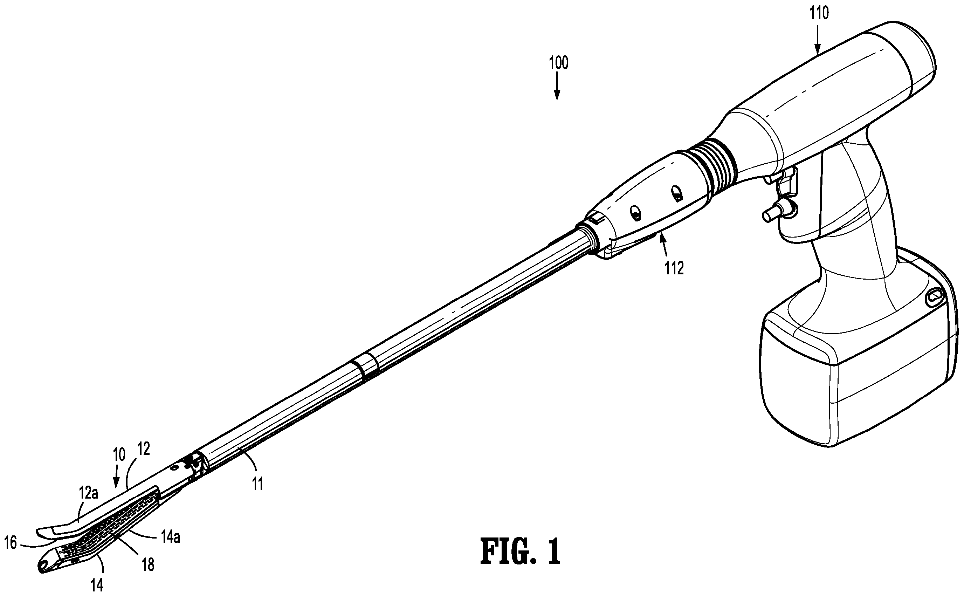

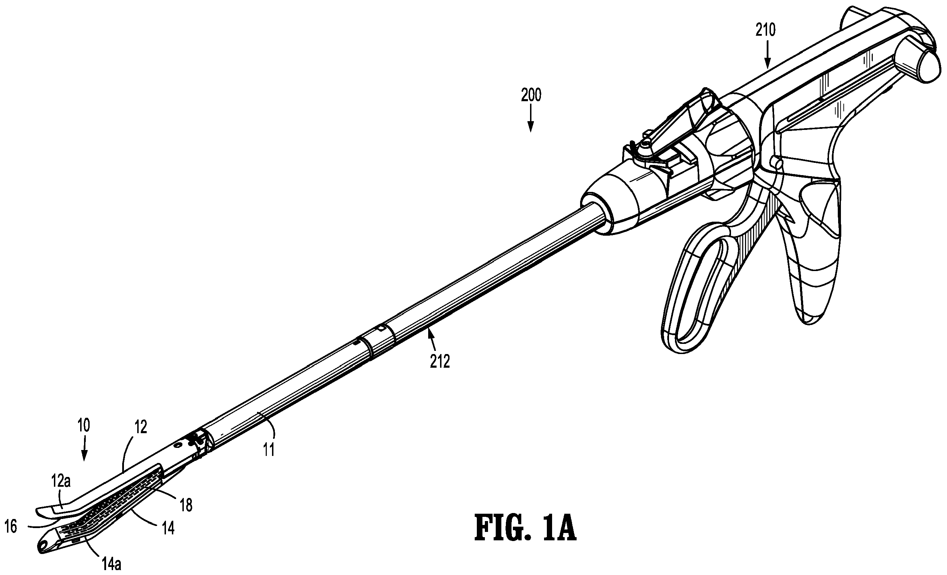

Referring to FIG. 1, the presently disclosed tool assembly shown generally as 10 is suitable for use with known surgical stapling devices. In embodiments, the surgical stapling device 100 includes a powered handpiece 110, an adapter 112 that extends distally from the powered handpiece 110, and the tool assembly 10 supported on the distal end of the adapter 112. The tool assembly 10 may be in the form of a disposable loading unit or reload 11 that is releasably coupled to the distal end of the adapter 112 using, for example, a bayonet-type coupling. Alternately, it is envisioned that the tool assembly 10, the adapter 112, and/or the powered handpiece may be integrally or non-removably secured to each other. U.S. Patent No. 9,055,943 ("'943 Patent") discloses a surgical stapling device having a powered handpiece, an adapter, and a tool assembly that is releasably coupled to the adapter.

Referring to FIG. 1A, in some embodiments, the surgical stapling device 200 includes a manually actuated hand piece 210, an elongated body portion 212 extending from the handpiece 210, and the tool assembly 10 coupled to the distal end of the elongated body portion 212. As discussed above with regard to the stapling device 100 (FIG. 1), The tool assembly 10 may be in the form of a disposable loading unit or reload 11 that is releasably coupled to the distal end of the elongated body portion 212 using, for example, a bayonet-type coupling. Alternately, it is envisioned that the tool assembly 10 and the elongated body portion 212 may be integrally or non-removably secured to each other. U.S. Patent No. 5,865,361("'361 Patent") discloses a surgical stapling device having a manually actuated handpiece, an elongated body portion, and a tool assembly that is releasably coupled to the elongated body portion. The staple cartridge can be attached to the loading unit or stapler jaw, or the staple cartridge can be part of a removable and replaceable cartridge assembly.

Referring to FIGS. 1-2, the tool assembly 10 includes a pair of jaws 12, 14 including an anvil 12a and a cartridge assembly 14a that are movable in relation to each other between spaced and approximated positions. In embodiments, one of the jaws 12, 14 may be fixed in relation to the adapter 112 and/or body portion 212 and the other jaw 12, 14 may be movably supported for movement between the spaced and approximated positions in relation to the one jaw member. Alternately, both of the jaws may be movable in relation to the adapter 112 and/or body portion 212 between the spaced and the approximated positions. The adapter 112 and the elongated body portion 212 can be rotatable with respect to powered handpiece 110/handpiece 210 along arrow AA1 (Fig. 1)/AA2 (Fig. 1A) to position the termination point along the opposite side. Depending on the surgical procedure and the location of the specimen side, this rotation can accommodate the surgeon.

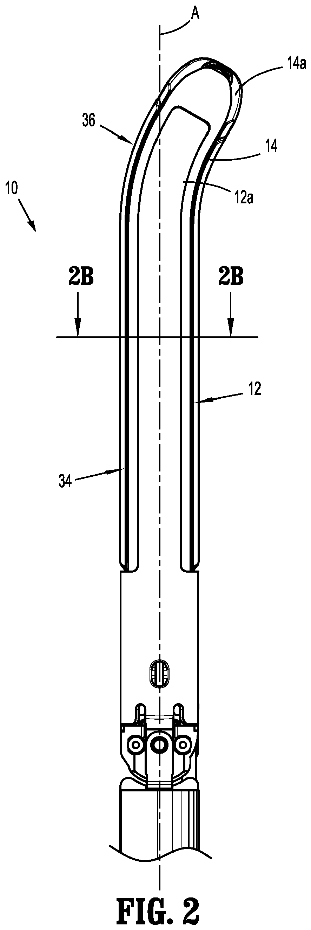

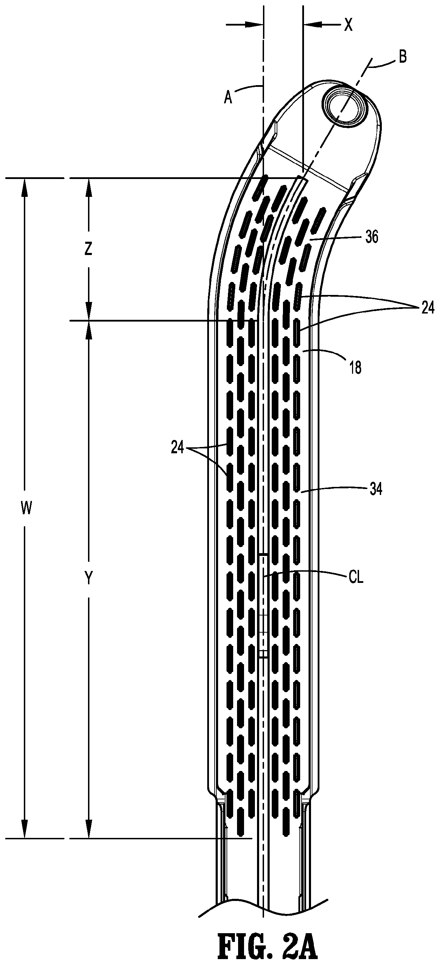

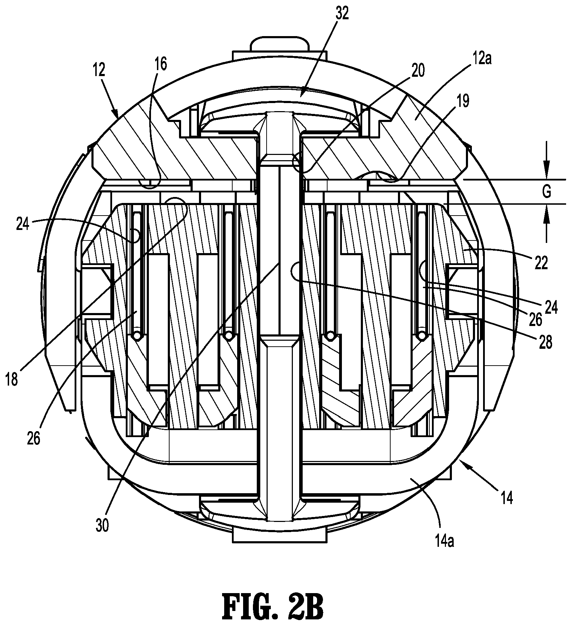

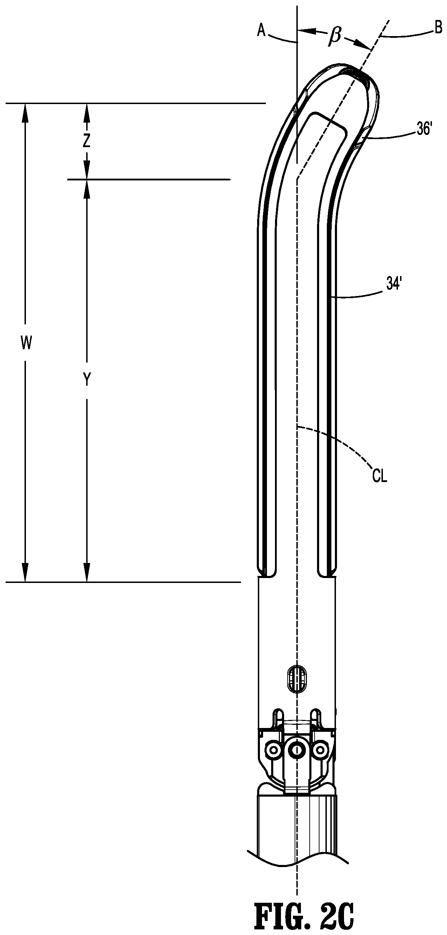

Referring to FIGS. 2-2B, each of the jaws 12, 14 defines a tissue contacting surface 16, 18, (FIG. 2B), respectively, that is positioned in opposition with the tissue contacting surface of the other jaw when the jaws 12, 14 are in the approximated position to define a tissue gap "G" (FIG. 2B). The tissue gap "G" is dimensioned to clamp onto tissue to be stapled and cut by the tool assembly 10 as is known in the art. The '361 Patent discloses a tool assembly configured to staple and cut tissue.

The tissue contacting surface 16 of the anvil 12a defines a plurality of staple deforming recesses 19 and an anvil knife slot 20 (FIG. 2B). In addition, the cartridge assembly 14a includes a staple cartridge 22 that defines staple pockets 24 that receive staples 26. The staple cartridge 22 also defines a knife slot 28 (FIG. 2B). The knife slots 20 and 28 are aligned and receive a knife 30 of a drive member 32 to cut tissue clamped between the anvil 12a and cartridge assemblies 14a as is known in the art. The '361 Patent discloses a tool assembly including a drive member and a knife. As such, these components will not be described in further detail herein.

The tool assembly 10 (and each of the jaw members 12, 14) includes a longitudinal portion 34 defining a first longitudinal axis "A" and a transverse portion 36 positioned contiguous with and distally of the longitudinal portion 34. The transverse portion 36 may be curved outwardly away from the first longitudinal axis "A" a distance "X" from the termination point TS (FIG. 2A). Without wishing to be bound to a particular theory, the distance "X" is spaced to ensure that the entire staple line (opposite the specimen side) is spaced to intersect the cut line. Stated another way, depending on the number of rows in the staple line (i.e. 2 rows, 3 rows), the cut line will contain at least a portion of all rows of the staple line on the non-specimen side. In one embodiment, the distance "X" may be between about 1mm and about 10mm. In other embodiments, the distance "X" may be between about 4mm and 8mm. In other embodiments, the distance "X" may be between 1mm and about 30mm and any subranges in between. In addition, the transverse portion 36 may have a radius of curvature to define a transition of the first longitudinal axis "A" and "B" The transverse portion may form a corner "C" between the linear portion and the transverse portion.

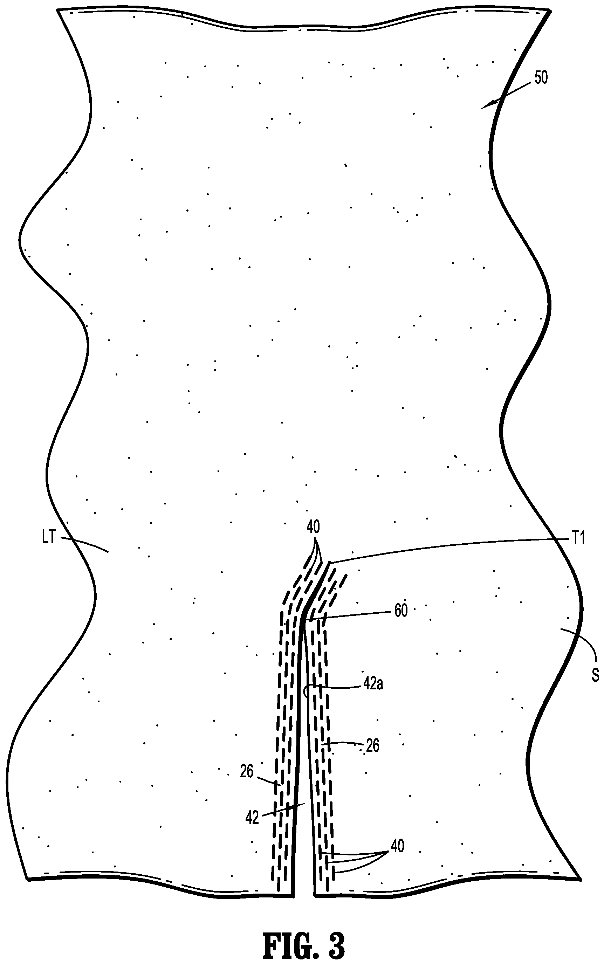

When actuated, an operative portion "W" of the tool assembly 10 defines staple lines 40 and a cut line 42 (FIG. 3) that extend along a substantial portion of the overall length of the tool assembly 10. As used herein, the operative portion "W" means the portion of the tool assembly 10 that forms the staple lines 40 and cut line 42. The longitudinal portion 34 of the operative portion "W" of the tool assembly 10 has a length "Y" and the transverse portion 36 has a length "Z". The length "Y" forms a majority of the overall length of the operative portion "W" of the tool assembly 10. In embodiments, the length "Y" is a greater percentage of the total length of the operative portion "W". According to the invention, the length "Y" can be at least 80 percent of the total length of the operative portion "W". In another embodiment, the length "Y" is at least 90 percent of the total length of the operative portion "W".

Referring to FIG. 2C, in an alternative embodiment, the transverse portion 36' of the tool assembly 10' is linear and defines a longitudinal axis "B" that defines an acute angle β with the axis "A" of the longitudinal portion 34'. In embodiments, angle β can have a value to ensure that the entire staple line (opposite the specimen side) is spaced to intersect the cut line. Stated another way, depending on the number of rows in the staple line (i.e. 2 rows, 3 rows), the cut line will contain at least a portion of all rows of the staple line on the non-specimen side. For example, angle β can be between about 15 degrees and about 60 degrees and in some embodiments is between about 20 degrees and 40 degrees and any subranges in between.

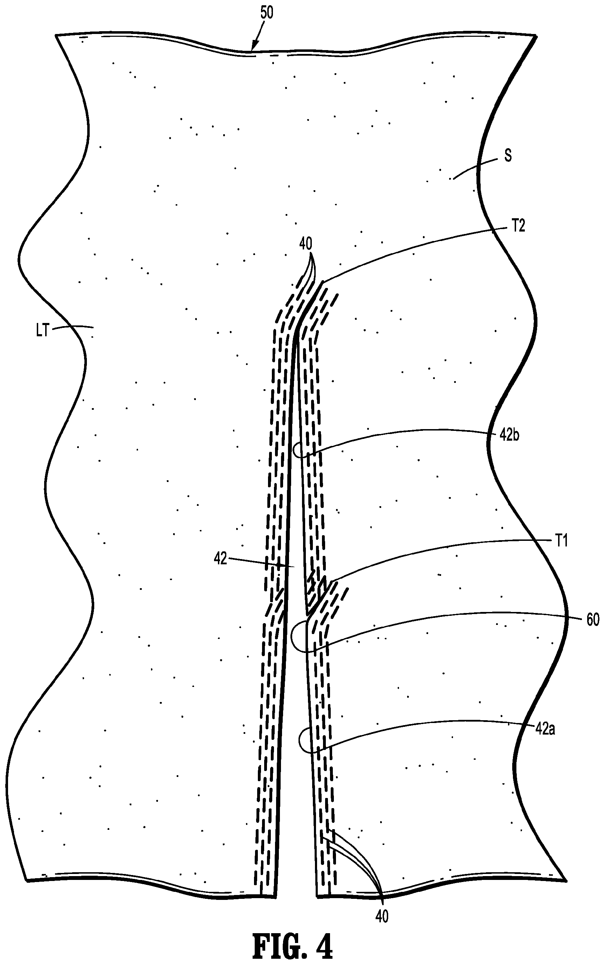

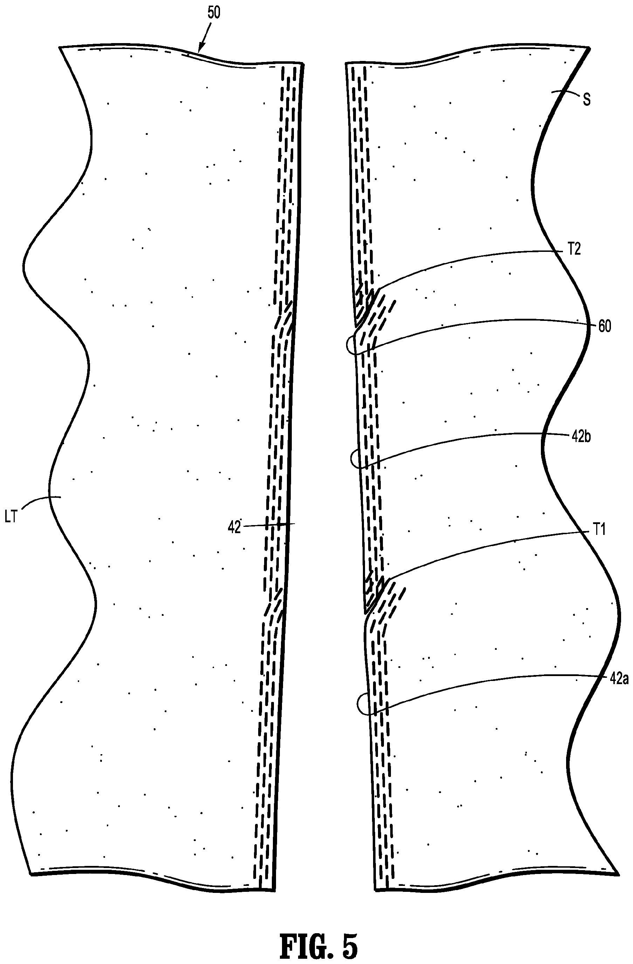

Referring to FIGS. 3-6, when a surgical procedure is performed that requires removal of tissue having a length greater than the length of the operative portion "W" (FIG. 2A) of the tool assembly 10, it is necessary to operate the tool assembly 10 a plurality of times to fully separate the tissue from the patient. Each time the tool assembly 10 is actuated to cut and staple tissue, a distal end of each cut line 42a, 42b, referred to herein as a termination points "T1" and "T2" (FIG. 4), may not be perfectly sealed and may be subject to leakage. This greater likelihood of leakage occurs at the termination points "T1" and "T2" because the tool assembly 10 does not place a staple transversely across the termination points "T1" and "T2". As described in further detail below, the presently disclosed tool assembly 10 allows a clinician to position the termination points "T1" and "T2" of a cut line 42 on the specimen side "S" of the tissue being operated upon to minimize the likelihood of leakage occurring in live tissue "LT" that remains within the patient.

In use, during a surgical procedure, e.g., a gastrectomy procedure, to remove tissue from a patient, e.g., a portion of the stomach 50, the jaws 12 and 14 (FIG. 1) of the tool assembly 10 are approximated about a portion of the stomach 50 and the surgical stapling device 100, 200 is actuated as known in the art to simultaneously cut and staple the tissue 50. Prior to cutting and stapling the tissue 50, a clinician positions the tool assembly 10 such that the transverse portion 36 of the tool assembly 10 angles or curves towards the specimen side "S" of the tissue 50 and the first termination point T1 of the first cut line 42a is positioned on the specimen side "S" of the tissue 50 offset from an axis defined by the cut line 42a.

After the first actuation of the surgical stapler 100, 200, the disposable unit 11 (FIGS. 1 and 1A) is replaced with a new disposable loading unit 11 including a fresh cartridge. Alternately, a new stapling device with a fresh cartridge can be used. Next, the stapling device 100, 200 is positioned such that the longitudinal portion 34 of the tool assembly 10 is aligned with the distal end 60 (FIG. 4) of the longitudinal portion of the existing cut line 42a and the transverse portion 36 of the tool assembly 10 is angled or curved outwardly from the longitudinal axis "A" towards the specimen side "S" of the tissue 50. When the stapling device 100, 200 is actuated a second time to staple tissue and form a second cut line 42b (FIG. 4), the first and second termination points T1 and T2 are positioned on the specimen side "S" of the tissue 50 offset from the longitudinal axis "A" of the first and second cut lines 42a and 42b or of at least the second cut line 42b if the cut line 42 (42a and 42b) is not straight.

If an additional actuation or actuations of the stapling device 100, 200 is/are required to fully dissect and remove the specimen side "S" of tissue 50 being operated on, the above-described step is repeated until the tissue is fully dissected and stapled and the tissue specimen "S" is removed. By performing the method described above with the presently disclosed tool assembly 10, all of the termination points on the cut line 42 are confined to the specimen side "S" of the tissue 50 being operated upon. As such, the points along the cut line 42 most susceptible to leakage are removed from the patient with the specimen side "S" of the tissue 50 after the dissection of tissue is complete.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A tool assembly (10) for leak resistant dissection of tissue comprising:
a cartridge assembly (14a) including a cartridge (22) defining a first longitudinal axis ("A") and having a linear longitudinal portion (34) and a transverse portion (36), the transverse portion (36) being contiguous with and positioned distally of the longitudinal portion (34), the cartridge (22) defining a central knife slot (28) and at least one row of staple receiving pockets (24) positioned on each side of the knife slot (28), wherein the central knife slot (28) and the at least one row of staple receiving pockets (24) extend along at least a portion of the transverse portion (36) of the cartridge body (14a); and
an anvil (12a) positioned along the first longitudinal axis ("A") having a linear longitudinal portion (34) and a transverse portion (36), the transverse portion (36) of the anvil (12a) being contiguous with and positioned distally of the longitudinal portion (34) of the anvil (12a), the anvil (12a) being supported adjacent to the cartridge assembly (14a) and defining an anvil knife slot (20) that is aligned with the central knife slot (28) of the cartridge body (22) and at least one row of staple deforming depressions (19) positioned on each side of the anvil knife slot (20), wherein the anvil knife slot (20) and the at least one row of staple deforming depressions (19) extend along at least a portion of the transverse portion (36) of the anvil (12a);
**characterised in that** the length ("Y") of the longitudinal portions (34) of the cartridge (14a) and the anvil (12a) is at least 80 percent of the overall length ("W") of the tool assembly (10).

2. The tool assembly (10) of any preceding claim, wherein the transverse portions (36) of the cartridge (14a) and the anvil (12a) are curvilinear.

3. The tool assembly (10) of claim 2, wherein the transverse portions (36) of the cartridge (14a) and anvil (12a) define a radius of curvature of between the linear longitudinal portion (34) and transverse portion (36).

4. The tool assembly (10) of any preceding claim, wherein the transverse portions (36) of the cartridge (14a) and anvil (12a) define a second longitudinal axis ("B"), the first longitudinal axis ("A") and the second longitudinal axis ("B") defining an angle β.

5. The tool assembly (10) of claim 4, wherein angle β is between 15 degrees and 60 degrees.

6. The tool assembly (10) of claim 5, wherein angle β is between 30 degrees and 45 degrees.

7. The tool assembly (10) of any preceding claim, wherein the distal end of the central knife slot (28) is spaced outwardly from the longitudinal axis ("A") a distance X, wherein X is between 2mm and 10mm.

8. The tool assembly (10) of claim 7, wherein X is between 4mm and 8mm.

9. The tool assembly (10) of any preceding claim, wherein the transverse portion (36) forms a corner between the transverse portion (36) and the longitudinal portion (34).

10. A surgical stapling device (100, 200) comprising a body portion (212) and the tool assembly (10) of any preceding claim, wherein the tool assembly (10) is coupled to the distal end of the body portion (212).

## Patentansprüche

1. Werkzeuganordnung (10) zur leckfreien Dissektion von Gewebe, die Folgendes umfasst:
eine Kartuschenanordnung (14a) mit einer Kartusche (22), die eine erste Längsachse ("A") definiert und einen linearen Längsabschnitt (34) aufweist, und
einen Querabschnitt (36), wobei der Querabschnitt (36) an den Längsabschnitt (34) angrenzt und distal davon positioniert ist, wobei die Kartusche (22) einen zentralen Messerschlitz (28) und mindestens eine Reihe von Klammeraufnahmetaschen (24) definiert, die auf jeder Seite des Messerschlitzes (28) positioniert sind, wobei sich der zentrale Messerschlitz (28) und die mindestens eine Reihe von Klammeraufnahmetaschen (24) entlang mindestens eines Abschnitts des Querabschnitts (36) des Kartuschenkörpers (14a) erstrecken; und
einen Amboss (12a), der entlang der ersten Längsachse ("A") mit einem linearen Längsabschnitt (34) und einem Querabschnitt (36) positioniert ist, wobei der Querabschnitt (36) des Ambosses (12a) an den Längsabschnitt (34) des Ambosses (12a) angrenzt und distal davon positioniert ist, wobei der Amboss (12a) angrenzend an die Kartuschenanordnung (14a) abgestützt ist und einen Ambossmesserschlitz (20) definiert, der auf den zentralen Messerschlitz (28) des Kartuschenkörpers (22) ausgerichtet ist, und mindestens eine Reihe von Klammerverformungsvertiefungen (19), die auf jeder Seite des Ambossmesserschlitzes (20) angeordnet sind, wobei sich der Ambossmesserschlitz (20) und die mindestens eine Reihe von Klammerverformungsvertiefungen (19) entlang mindestens eines Teils des Querabschnitts (36) des Ambosses (12a) erstrecken;
**dadurch gekennzeichnet, dass** die Länge ("Y") der Längsabschnitte (34) der Kartusche (14a) und des Ambosses (12a) mindestens 80 Prozent der Gesamtlänge ("W") der Werkzeuganordnung (10) beträgt.

2. Werkzeuganordnung (10) nach einem der vorhergehenden Ansprüche, wobei die Querabschnitte (36) der Kartusche (14a) und des Ambosses (12a) krummlinig sind.

3. Werkzeuganordnung (10) nach Anspruch 2, wobei die Querabschnitte (36) der Kartusche (14a) und des Ambosses (12a) einen Krümmungsradius zwischen dem linearen Längsabschnitt (34) und dem Querabschnitt (36) definieren.

4. Werkzeuganordnung (10) nach einem der vorhergehenden Ansprüche, wobei die Querabschnitte (36) der Kartusche (14a) und des Ambosses (12a) eine zweite Längsachse ("B") definieren, wobei die erste Längsachse ("A") Und die zweite Längsachse ("B") einen Winkel β definieren.

5. Werkzeuganordnung (10) nach Anspruch 4, wobei der Winkel β zwischen 15 Grad und 60 Grad liegt.

6. Werkzeuganordnung (10) nach Anspruch 5, wobei der Winkel β zwischen 30 Grad und 45 Grad liegt.

7. Werkzeuganordnung (10) nach einem vorhergehenden Anspruch, wobei das distale Ende des zentralen Messerschlitzes (28) nach außen von der Längsachse ("A") um einen Abstand X beabstandet ist, wobei X zwischen 2 mm und 10 mm beträgt.

8. Werkzeuganordnung (10) nach Anspruch 7, wobei X zwischen 4 mm und 8 mm beträgt.

9. Werkzeuganordnung (10) nach einem vorhergehenden Anspruch, wobei der Querabschnitt (36) eine Ecke zwischen dem Querabschnitt (36) und dem Längsabschnitt (34) bildet.

10. Chirurgische Klammervorrichtung (100, 200), die einen Körperabschnitt (212) und die Werkzeuganordnung (10) nach einem vorhergehenden Anspruch umfasst, wobei die Werkzeuganordnung (10) mit dem distalen Ende des Körperabschnitts (212) gekoppelt ist.

## Revendications

1. Ensemble outil (10) pour une dissection résistante aux fuites d'un tissu, comprenant :
un ensemble cartouche (14a) comportant une cartouche (22) définissant un premier axe longitudinal (« A ») et ayant une partie longitudinale linéaire (34) et
une partie transversale (36), la partie transversale (36) étant contiguë et positionnée distalement à la partie longitudinale (34), la cartouche (22) définissant une fente à couteau centrale (28) et au moins une rangée de poches de réception d'agrafes (24) positionnées de chaque côté de la fente à couteau (28), la fente à couteau centrale (28)et l'au moins une rangée de poches de réception d'agrafes (24) s'étendant le long d'au moins une partie de la partie transversale (36) du corps de cartouche (14a) ; et
une enclume (12a) positionnée le long du premier axe longitudinal (« A ») ayant une partie longitudinale linéaire (34) et une partie transversale (36), la partie transversale (36) de l'enclume (12a) étant contiguë et positionnée distalement à la partie longitudinale (34) de l'enclume (12a), l'enclume (12a) étant supportée dans une position adjacente à l'ensemble cartouche (14a) et définissant une fente à couteau de l'enclume (20) qui est alignée avec la fente à couteau centrale (28) du corps de cartouche (22) et au moins une rangée de dépressions de déformation d'agrafes (19) positionnées de chaque côté de la fente à couteau de l'enclume (20), la fente à couteau de l'enclume (20) et au moins une rangée de dépressions de déformation d'agrafes (19) s'étendant le long d'au moins une partie de la partie transversale (36) de l'enclume (12a) ;
**caractérisé en ce que** la longueur (« Y ») des parties longitudinales (34) de la cartouche (14a) et de l'enclume (12a) représente au moins 80 % de la longueur totale (« W ») de l'ensemble outil (10).

2. Ensemble outil (10) selon l'une quelconque des revendications précédentes, dans lequel les parties transversales (36) de la cartouche (14a) et l'enclume (12a) sont curvilignes.

3. Ensemble outil (10) selon la revendication 2, dans lequel les parties transversales (36) de la cartouche (14a) et de l'enclume (12a) définissent un rayon de courbure entre la partie longitudinale linéaire (34) et la partie transversale (36).

4. Ensemble outil (10) selon l'une quelconque des revendications précédentes, dans lequel les parties transversales (36) de la cartouche (14a) et de l'enclume (12a) définissent un second axe longitudinal (« B »), le premier axe longitudinal (« A ») et le second axe longitudinal (« B ») définissant un angle β.

5. Ensemble outil (10) selon la revendication 4, dans lequel l'angle β est compris entre 15 degrés et 60 degrés.

6. Ensemble outil (10) selon la revendication 5, dans lequel l'angle β est compris entre 30 degrés et 45 degrés.

7. Ensemble outil (10) selon l'une quelconque des revendications précédentes, dans lequel l'extrémité distale de la fente à couteau centrale (28) est espacée vers l'extérieur de l'axe longitudinal (« A ») d'une distance X, X étant compris entre 2 mm et 10 mm.

8. Ensemble outil (10) selon la revendication 7, dans lequel X est compris entre 4 mm et 8 mm.

9. Ensemble outil (10) selon l'une quelconque des revendications précédentes, dans lequel la partie transversale (36) forme un coin entre la partie transversale (36) et la partie longitudinale (34).

10. Dispositif d'agrafage chirurgical (100, 200) comprenant une partie corps (212) et l'ensemble outil (10) selon l'une quelconque des revendications précédentes, dans lequel l'ensemble outil (10) est accouplé à l'extrémité distale de la partie corps (212).
